# EUROPEAN PATENT APPLICATION

(11) **EP 4 681 555 A2**
(43) Date of publication of application: **21.01.2026**
(21) Application number: 25219794.2
(22) Date of filing: 11.10.2022
(51) Int. Cl.: A23L 33/20

(54) **PROCESS FOR MODIFYING GLUCO-OLIGOSACCHARIDES**

(30) Priority: 12.10.2021 EP 21202110
(62) Divisional of application: 22798038.0
(71) Applicant: Cargill, Incorporated, Wayzata, Minnesota 55391 (US)
(72) Inventor: De Doncker, Marc, Antwerpen (BE); Desmet, Tom, Nevele (BE); Vercauteren, Ronny Leontina Marcel, Haasdonk (BE)
(74) Representative: Cargill IP Department

(57) **Abstract**

The present invention relates to an enzymatic process for elongating gluco-oligosaccharides with a glucosyl moiety. The invention also relates to the elongated gluco-oligosaccharides obtained with the process. These elongated gluco-oligosaccharides can be used in edible products, for instance as sweeteners and/or (low calorie) bulking agents and present a decreased digestibility compared to known gluco-oligosaccharides.

## Description

### CROSS-REFERENCE TO RELATED APPLICATIONS

This application claims the benefit of European Patent Application No. 21202110.9, filed October 12, 2021, which is incorporated by reference herein in its entirety.

### SEQUENCE LISTING

The content of the Sequence Listing XML of the sequence listing named "PT-00782-US-PCT.xml" which is 10.2kb in size created on October 11, 2022 and electronically submitted via Patent Center herewith the application is incorporated by reference in its entirety.

### BACKGROUND

The present invention relates to an enzymatic process for elongating gluco-oligosaccharides with a glucosyl moiety. The invention also relates to the elongated gluco-oligosaccharides obtained with the process. These elongated gluco-oligosaccharides can be used in edible products, for instance as a dietary fiber or a (low calorie) bulking agent or a prebiotic or in sugar replacement or in calorie reduction, and present a decreased digestibility compared to known gluco-oligosaccharides.

### FIELD OF THE INVENTION

Oligo- and polysaccharides are widely used in the food industry. Hydrolyzed plant-based starches are a source of glucose containing oligo- and polysaccharides. Hydrolyzed starches include maltodextrins and glucose syrups.

Maltodextrins and glucose syrups may be used as a as a texturizer or a (full caloric) ingredient also providing bulking functionality, or as a coating and for encapsulation. Maltodextrins and glucose syrups are easily digested and are fully and rapidly absorbed by the gastrointestinal tract. The high and fast digestibility results in a strong rise in blood glucose levels after consumption. Maltodextrins and glucose syrups are therefore products with a high glycemic index. Further, maltodextrins and glucose syrups are fully caloric carbohydrates (4 kcal/g).

Currently, there is a need in the food industry for bulking ingredients and carbohydrates that have a lower or slower digestibility, are lower in calories, and to decrease the glycemic impact of the oligo- or polysaccharides used for this purpose. One suggestion has been to replace the existing polysaccharides and carbohydrates with naturally occurring alternatives with a lower calorie content. Another alternative is to modify existing oligo- and polysaccharides.

The present inventors have now found a process for modifying oligo- or polysaccharides such as maltodextrins or glucose syrups by means of an enzymatic process, resulting in a carbohydrate with decreased digestibility.

### STATEMENTS OF THE INVENTION

The present invention thus provides in a first aspect a process for elongating gluco-oligosaccharides with at least one α-(1,2)-terminally linked glucosyl moiety, comprising the steps of:
providing an acceptor composition comprising gluco-oligosaccharides having a degree of polymerization of 2, 3, 4, 5 or 6 glucosyl moieties, wherein each gluco-oligosaccharide comprises at least one α-(1,4)-linkage and wherein the acceptor composition comprises at least two gluco-oligosaccharides having a different degree of polymerization;
placing the acceptor composition in fluid contact with a polypeptide having kojibiose phosphorylase activity in the presence of a 1-glucosyl phosphate donor.

The present invention results in the addition of one or more glucosyl moieties to the acceptor gluco-oligosaccharide introducing an α-1,2-linkage between the glucosyl moiety and the gluco-oligosaccharide. The glucosyl moiety is terminally linked to the gluco-oligosaccharide, *i.e.,* it is bound to the non-reducing end of the gluco-oligosaccharide.

Without wishing to be bound to any theory, it is believed that the orientation of this linkage leads to steric hindrance of the digestive enzymes in the gastrointestinal tract.

The use of polypeptide having kojibiose phosphorylase activity is known. For instance, US6066477 describes a kojibiose phosphorylase that catalyzes the transfer reaction of a glucosyl group to a single monosaccharide or to a single oligosaccharide, using β-D-glucose-1-phosphoric acid as a saccharide donor. The enzyme is obtainable from natural sources such as microorganisms of the genus *Thermoanaerobium* or by recombinant technology.

Chaen et al, Journal of Bioscience and bioengineering, Vol. 92, No. 2, 173-176, 2001, describe the enzymatic synthesis of oligosaccharides from L-sorbose, maltose and sucrose using kojibiose phosphorylase.

The inventors have now found that using an acceptor composition which is a mixture of gluco-oligosaccharides having different degrees of polymerization, in particular a hydrolyzed starch, elongation of the gluco-oligosaccharides with (at least one) α-1,2-linked glucosyl moiety can be obtained, resulting in a gluco-oligosaccharide composition that has decreased digestibility.

This result is unexpected since it was uncertain which behavior the polypeptide having kojibiose phosphorylase activity would show towards the mixture of these non-native acceptor substrates, as no experiments involving a mixture of two or more gluco-oligosaccharides have been reported to date. It could be expected that certain gluco-oligosaccharides, e.g., with higher degree of polymerization, are only marginally glucosylated by kojibiose phosphorylase in the presence of other gluco-oligosaccharides, e.g., with lower degree of polymerization, as these enzymes are known to distinguish between substrates with different degrees of polymerization.

Thus, it was expected that the polypeptide having kojibiose phosphorylase activity would be selective for one gluco-oligosaccharide, or for a group of gluco-oligosaccharides with the smallest degree of polymerization. It has now been found that in a mixture of gluco-oligosaccharides with different degrees of polymerization, each gluco-oligosaccharide is elongated to the same extend by the kojibiose phosphorylase activity, i.e., the conversion rate of each gluco-oligosaccharide from the acceptor composition to the elongated gluco-oligosaccharide is the same or similar.

In particular the conversion rate is expressed as the molar ratio [elongated gluco-oligosaccharide]/[acceptor oligosaccharide] for each degree of polymerization. This molar ratio is from 35/65 to 45/55. This ratio can be determined on the basis of the peak areas of the HPAEC chromatogram, as will be shown later in the experimental section.

In a second aspect, the invention relates to a composition comprising gluco-oligosaccharides elongated with at least one α-(1,2)-terminally linked glucosyl residue.

In a third aspect, the invention relates to an edible product comprising the composition of the invention.

### DETAILED DESCRIPTION

### Process

### Acceptor composition

As described above, the process of the invention has as a starting material an acceptor composition comprising gluco-oligosaccharides having a degree of polymerization of 2, 3, 4, 5 or 6 glucosyl moieties, wherein each gluco-oligosaccharide comprises at least one α-(1,4)-linkage and wherein the acceptor composition comprises at least two gluco-oligosaccharides having a different degree of polymerization.

With gluco-oligosaccharides are meant oligosaccharides having 2 or more glucosyl moieties in the molecular chain. These glucosyl moieties can be bound to each other via α-(1,4)-linkages or via α-(1,6)-linkages. In the starting gluco-oligosaccharides of the invention, at least one α-(1,4)-linkage is present.

The acceptor composition comprises at least two gluco-oligosaccharides having a different degree of polymerization. For instance, the composition may comprise a gluco-oligosaccharide having a degree of polymerization of 2 (two glucosyl moieties) and a gluco-oligosaccharide having a degree of polymerization of 3 (three glucosyl moieties).

The acceptor composition of the invention can be obtained by mixing 2, 3, 4 or more gluco-oligosaccharides having different degrees of polymerization. Alternatively, the acceptor composition of the invention can be obtained by partial hydrolysis of starch as will be described hereafter.

Preferably, the acceptor composition comprises at least two different gluco-oligosaccharides selected from the group consisting of maltose, maltotriose, maltotetraose, maltopentaose and maltohexaose. CAS numbers for these compounds are: Maltose: 69-79-4; Maltotriose 1109-28-0; Maltotetraose 34612-38-9; Maltopentaose 34620-76-3; Maltohexaose 34620-77-4.

Beside the gluco-oligosaccharides having a degree of polymerization of 2, 3, 4, 5 or 6 glucosyl moieties the acceptor composition can comprise further oligo- or polysaccharides, for instance those present in an acceptor composition obtained by partial hydrolysis of starch.

The acceptor composition is preferably a partially hydrolyzed starch having a dextrose equivalent (DE) of less than 60, preferably less than 50, more preferably less than 45. The starch hydrolysate has a DE of at least 4, preferably at least 10.

The partially hydrolyzed starch may be obtained by submitting starch to enzymatic or acid hydrolysis or a combination thereof, such as acid hydrolysis followed by enzyme hydrolysis.

Dextrose equivalent (DE) is a measure of the total reducing sugars calculated as D-glucose on a dry weight basis. The approved method for determining DE is the Lane-Eynon titration method, which measures reduction of a copper sulfate solution. Unhydrolyzed starch has a DE value of zero, while the DE value of anhydrous D-glucose is 100. Glucose syrups range from 20 to 97 DE.

Maltodextrins are the dried products or aqueous solutions of saccharides obtained from edible starch having a dextrose equivalent of less than 20. Glucose syrups are aqueous solutions of nutritive saccharides obtained from edible starch having a dextrose equivalent of 20 or more. Fructose syrups are aqueous solutions of nutritive saccharides obtained from edible starch in which a portion of the dextrose (D-glucose) has been isomerized to fructose.

When a partially hydrolyzed starch is used as the acceptor composition, it may contain further gluco-oligosaccharides having α-1,6-linkages, such as the isoforms of maltotriose, maltotetraose, maltopentaose and maltohexaose and oligosaccharides containing mixed α-1,6 and α-1,4 linkages and oligosaccharides having glucopyranosyl branches through mixed α-1,6 and α-1,4 linkages. The acceptor composition may also comprise gluco-oligosaccharides having a degree of polymerization higher than 6, e.g., 7 or 8.

### Polypeptide

Polypeptides with kojibiose phosphorylase activity are known and are enzymes classified under E.C.2.4.1.230. Kojibiose phosphorylase (KP) is a carbohydrate-processing phosphorolytic enzyme and is classified into glycoside hydrolase family 65 (GH65). KP catalyzes the reversible phosphorolysis of kojibiose (α-D-glucopyranosyl-(1→2)-D-glucopyranose) to β-D-glucose-1-phosphate (β-G1P or beta-G1P) and D-glucose (Glc), with an inversion of the anomeric configuration. KP activity was first identified in cell extract of *Thermoanaerobacter brockii* ATCC35047. In the presence of suitable acceptors, KP from this strain (Tb-KP) transfers a glucosyl moiety from β-G1P to the acceptors, and synthesizes kojioligosaccharides. (Takuo Yamamote et. al., Biosci. Biotechnol. Biochem., 75 (6), 1208-1210, 2011*).*

The polypeptide includes any one of those separated from natural resources such as cultures of microorganisms which produce the polypeptide, mutants thereof obtained by treating with mutagens, and those which are artificially synthesized by applying recombinant DNA and peptide-synthesizing technologies. Preferably, the polypeptide having kojibiose phosphorylase activity is a recombinant kojibiose phosphorylase.

The recombinant kojibiose phosphorylase is preferably a polypeptide obtainable from a bacterium selected from the group consisting of *Thermococcus barophilus, Pseudothermotoga thermarum, Thermoanaerobacter brockii, Thermoanaerobacterium thermosacharolyticum, Caldicellulosiruptor saccharolyticus, Pyrococcus sp., Palaeococcus pacificus* and *Thermofilum pendens.*

According to the present invention the polypeptide is able to elongate gluco-oligosaccharides with at least one α-(1,2)-terminally linked glucosyl moiety, wherein the glucosyl moiety is added adjacent to an α-(1,4)-linkage present in said gluco-oligosaccharide.

### Donor

As described above, the donor is 1-glucosyl phosphate donor. Preferably, the 1-glucosyl phosphate donor is β-D-glucose-1-phosphate. β-D-glucose-1-phosphate is commercially available and can be used as such.

### Process coupled reaction

The donor can also be prepared by contacting an appropriate phosphorylase with a saccharide as a substrate in the presence of phosphoric acid or a salt thereof. Thus, according to a first aspect of this coupled reaction, the present invention preferably comprises a step of preparing 1-glucosyl phosphate donor by placing maltose in fluid contact with a polypeptide having maltose phosphorylase activity and phosphoric acid, a salt thereof or a combination thereof.

If the acceptor composition is partially hydrolyzed starch, the acceptor composition may already contain the maltose, or the maltose is generated *in situ* during the α-1,2-linkage-creating reactions.

The polypeptide comprising maltose phosphorylase activity is an enzyme that catalyzes the reaction of maltose and inorganic phosphate to glucose and β-D-glucose-1-phosphate. It is classified under EC 2.4.1.8. In particular the polypeptide comprising maltose phosphorylase activity is a recombinant maltose phosphorylase, preferably a polypeptide obtainable from a Bacterium selected from the group consisting of *Lactobacillus acidophilus, Bacillus selenitireducens, Enterococcus faecalis, Lactobacillus brevis, Lactobacillus sanfranciscensis, Paenibacillus sp. and Bacillus sp..*

An example is a maltose phosphorylase from *Lactobaciluus acidophilus,* such as described by Nakai, H., et al. (2009), The maltodextrin transport system and metabolism in Lactobacillus acidophilus NCFM and production of novel α-glucosides through reverse phosphorolysis by maltose phosphorylase. The FEBS Journal, 276: 7353-7365.

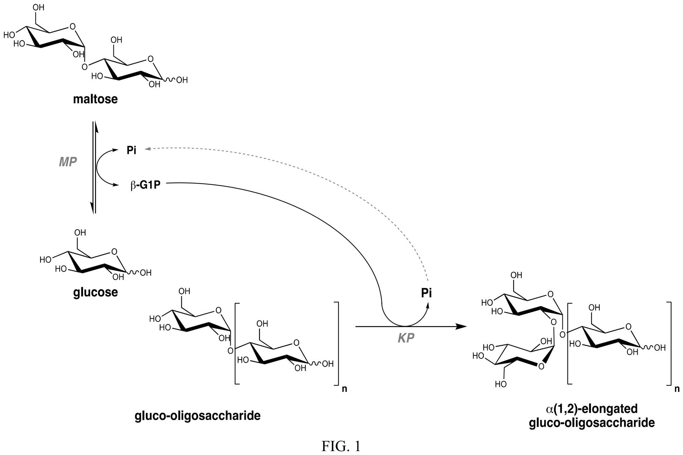

This "coupled reaction" is represented in Figure 1. In Figure 1, MP represents maltose phosphorylase from *Lactobacillus acidophilus* (LaMP), Pi stands for phosphoric acid and/or salt, β-G1P is β-D-glucose-1-phosphate, and KP represents kojibiose phosphorylase.

According to an aspect of the coupled reaction, the present invention preferably comprises a step of preparing 1-glucosyl phosphate donor by placing trehalose in fluid contact with a polypeptide having trehalose phosphorylase activity, and phosphoric acid and/or a salt thereof.

The polypeptide comprising trehalose phosphorylase activity is a polypeptide that catalyzes the reaction of trehalose and inorganic phosphate to glucose and β-D-glucose-1-phosphate. It is classified under (EC 2.4.1.64). See for instance TRENDS in Biotechnology Vol.20 No.10 October 2002.

### Process conditions

The process of the invention is carried out by placing the acceptor composition and donor in fluid contact with the polypeptide. In general, this means that the process is carried out in an aqueous solution. The polypeptide can also be an immobilized polypeptide.

Process conditions are those generally known in the field. For illustration, concentration of acceptor composition in the aqueous solution is from 5 to 60 wt.%; the pH of the reaction is from 4 to 7 (such as from 5 to 7); the molar ratio acceptor composition to donor composition is from 3:1 to 0.5: 1, the temperature is from 40 to 70 °C; the concentration of the polypeptide is 0.2 Units/mL.

It is also possible to include further polypeptides in the reaction, either simultaneously or sequentially with the process of the invention, e.g., alpha-amylase (including variations such as maltogenic alpha-amylase) and/or beta-amylase and/or additional debranching enzymes (pullulanase or/and isoamylase).

The process can further comprise a step of purifying the composition obtained such as by filtration and/or microfiltration, decolorization/deodorization using active carbon or porous polymeric resins (like styrene divinyl benzene resins) and/or demineralization using ion exchange resins. Also, nano- or ultrafiltration and/or chromatographic separation steps can be carried out to fractionate the composition or to eliminate smaller mono- and oligosaccharides from the composition.

### Product

The invention also relates to the composition comprising elongated gluco-oligosaccharides as obtained by the process described above.

The composition of the invention comprises gluco-oligosaccharides elongated with at least one α-(1,2)-terminally linked glucosyl moiety, wherein the gluco-oligosaccharides have a degree of polymerization of 2, 3, 4, 5 or 6, or even 7, 8, or 9; and wherein the composition comprises at least two gluco-oligosaccharides having a different degree of polymerization and wherein each gluco-oligosaccharide comprises at least one α-(1,4)-linkage. The elongated gluco-oligosaccharides according to the present invention preferably comprise at least one α-(1,2)-terminally linked glucosyl moiety that is adjacent to an α-(1,4)-linkage present in said gluco-oligosaccharide.

With the process of the invention, it is also possible to obtain a product where two or three glucosyl moieties are linked to the gluco-oligosaccharide, the first to the terminal non-reducing end of the gluco-oligosaccharide, the second to the previously attached glucosyl moiety.

In particular the invention also covers a composition which comprises two or more different elongated gluco-oligosaccharides with the following formulas: 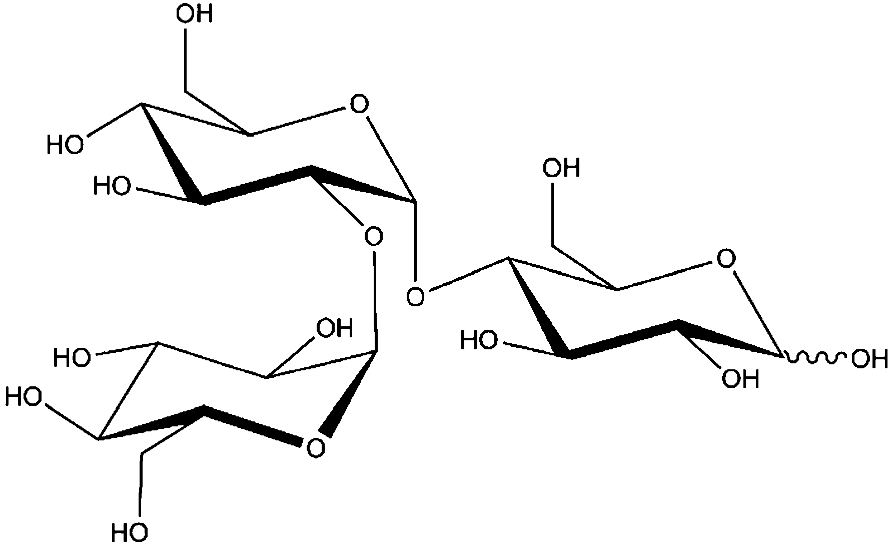 2-O-α-D-glucosyl-maltose
*i.e.,* α-D-Glucopyranosyl-(1→2)-α-D-glucopyranosyl-(1→4)-D-glucopyranose 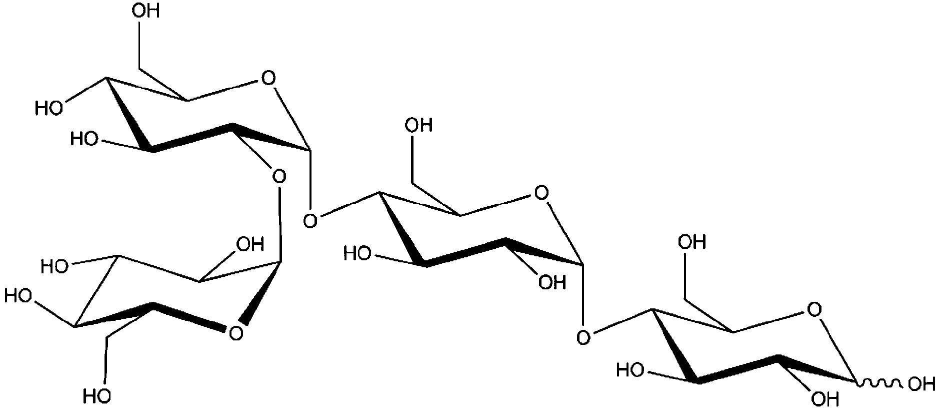 2-O-α-D-glucosyl-maltotriose
*i.e.,* α-D-Glucopyranosyl-(1→2)-α-D-glucopyranosyl-(1→4)-α-D-glucopyranosyl-(1→4)-D-glucopyranose 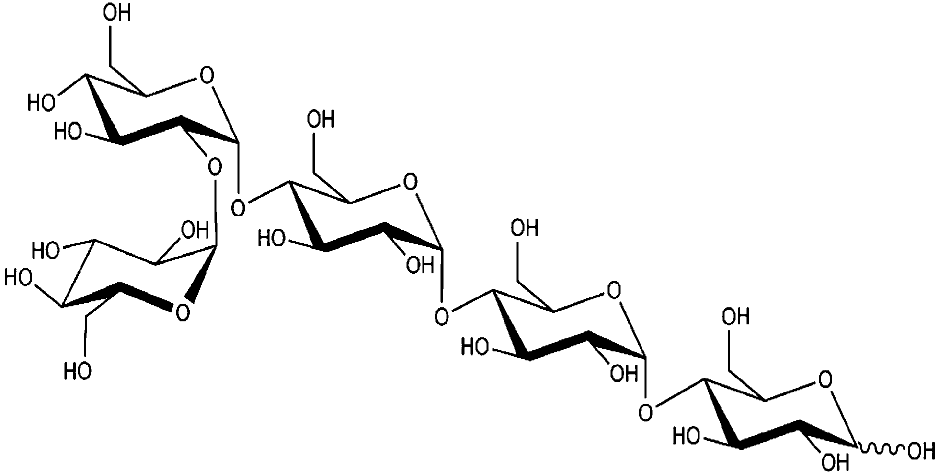 2-O-α-D-glucosyl-maltotetraose
*i.e.,* α-D-Glucopyranosyl-(1→2)-α-D-glucopyranosyl-(1→4)-α-D-glucopyranosyl-(1→4)-α-D-glucopyranosyl-(1→4)-D-glucopyranose 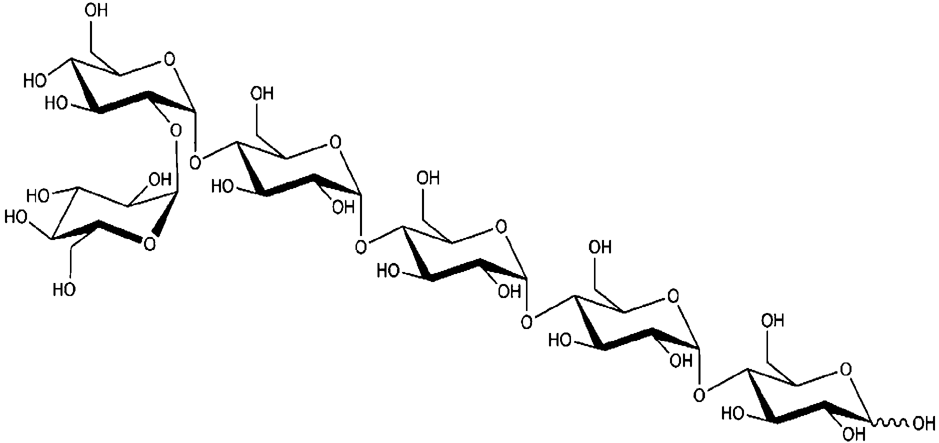 2-O-α-D-glucosyl-maltopentaose
*i.e.,* α-D-Glucopyranosyl-(1→2)-α-D-glucopyranosyl-(1→4)-α-D-glucopyranosyl-(1→4)-α-D-glucopyranosyl-(1→4)-α-D-glucopyranosyl-(1→4)-D-glucopyranose 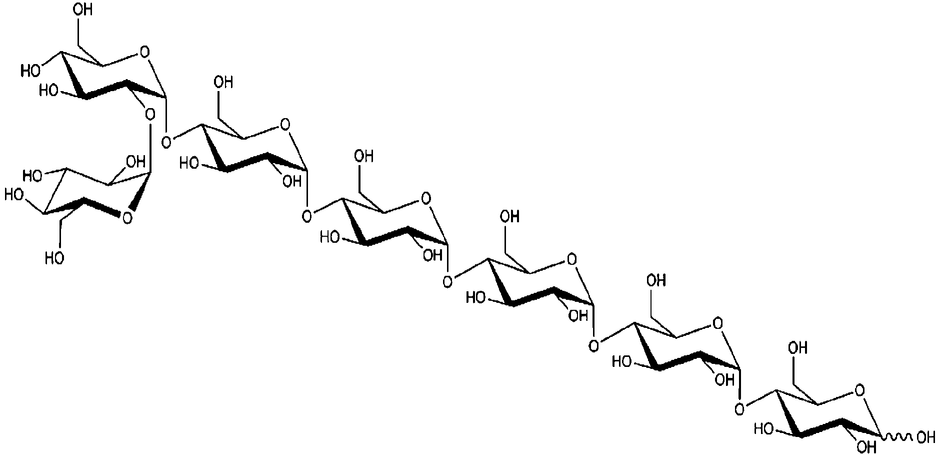 2-O-α-D-glucosyl-maltohexaose
*i.e.,* α-D-Glucopyranosyl-(1→2)-α-D-glucopyranosyl-(1→4)-α-D-glucopyranosyl-(1→4)-α-D-glucopyranosyl-(1→4)-α-D-glucopyranosyl-(1→4)-α-D-glucopyranosyl-(1→4)-D-glucopyranose

In particular, the composition of the invention is characterized by elongated gluco-oligosaccharides comprising saccharide linkages selected from α-(1,2)-; α-(1,4)-and other saccharide linkages; wherein the amount of α-(1,4)-linkages is more than 50% and the amount of α-(1,2)-linkages is less than 50% of all saccharide linkages, excluding linkages from kojibiose and kojioligosaccharides.

The ratio of α-(1,4)-linkages and α-(1,2)-linkages can be determined by high performance anion exchange chromatography (HPAEC), and/or NMR techniques and/or structural analysis by methylation.

The composition may further contain kojibiose and kojioligosaccharides. These compounds also contribute to decreased digestibility of the composition of the invention.

Kojibiose and kojioligosaccharides are in particular characterized by the following formulas: 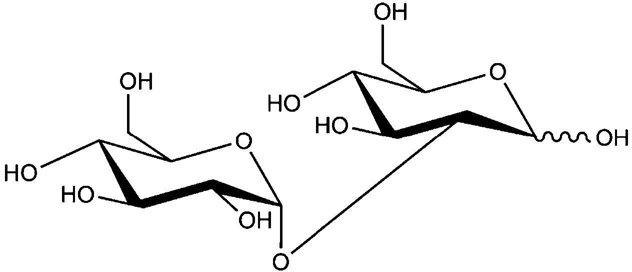 kojibiose
*i.e.,* α-D-Glucopyranosyl-(1→2)-D-glucopyranose 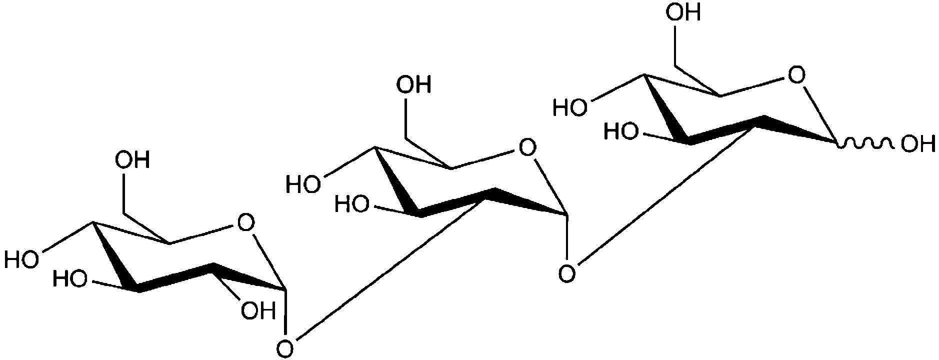 kojitriose
*i.e.,* α-D-Glucopyranosyl-(1→2)-α-D-glucopyranosyl-(1→2)-D-glucopyranose 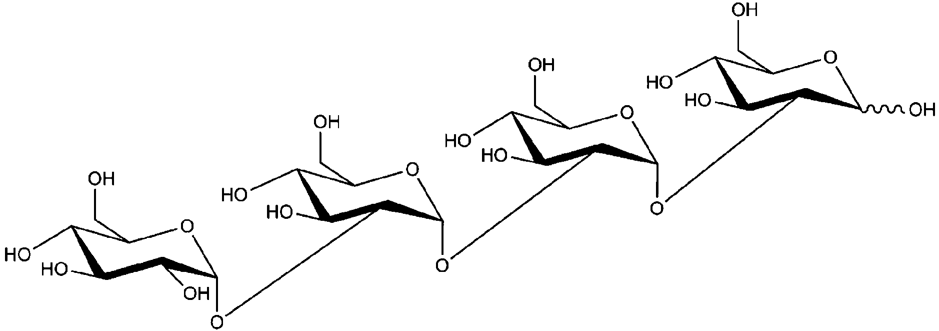 kojitetraose
*i.e.,* α-D-Glucopyranosyl-(1→2)-α-D-glucopyranosyl-(1→2)-α-D-glucopyranosyl-(1→2)-D-glucopyranose 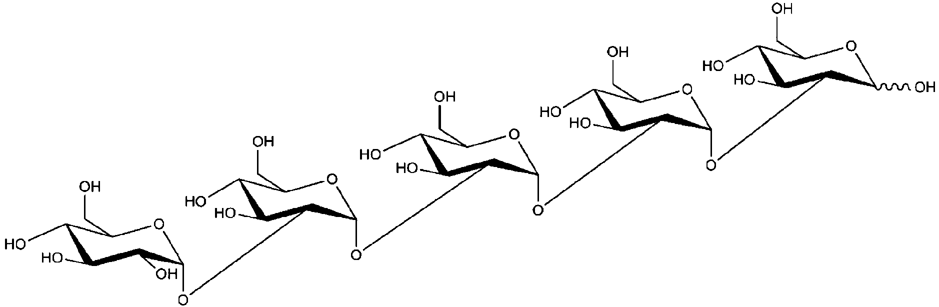 kojipentaose
*i.e.,* α-D-Glucopyranosyl-(1→2)-α-D-glucopyranosyl-(1→2)-α-D-glucopyranosyl-(1→2)-α-D-glucopyranosyl-(1→2)-D-glucopyranose

In particular the product of the invention comprises
- at least 20 wt.% elongated gluco-oligosaccharides
- at least 15 wt.% kojioligosaccharides
based on the total weight of the product as dry substance.

The product contains preferably less than 20 wt.% free glucose and fructose.

### Digestibility

Digestibility of the product can be shown by *in-vitro* digestion tests using in a first stage an alpha-amylase, followed by a digestion stage using a glucoamylase, an alpha-glucosidase or intestinal animal enzymes. Both stages can be combined to one digestion reaction using alpha-amylase and a glucoamylase or an alpha-glucosidase and intestinal animal enzymes.

Other advantageous properties of the product besides the lower calories compared to sucrose, are a high water solubility, viscosities comparable to commercial glucose syrups and maltodextrins and a lower relative sweetness than sucrose.

### Use of the composition obtained

The composition of the invention can be used in the form of a syrup or it can be dried down to be used in the form of a powder.

The composition of the invention can be used in foodstuffs for human consumption. In particular it can be used as a sweetener, sugar replacer, (low calorie) bulking agent, fiber, low calorie carbohydrate composition, prebiotic composition, low glycemic carbohydrate composition, a starch retrograding inhibiting agent, a crystallization inhibiting agent, an osmotic pressure regulator or a water activity regulator. In particular it can be used for calorie and/or sugar reduction.

The composition of the invention can be used in foodstuffs in combination with other bulking agents, fillers, soluble fibers, resistant starch, polydextrose, dextrins, resistant maltodextrin, inulin, or insoluble fibers. The combination can boost the fiber content of the foodstuff, enhance physiological benefits from consumption of the foodstuff, reduce the calorie content, reduce the sugars content, and/or enhance the nutritional profile of the foodstuff.

The composition of the invention can also be used in foodstuffs in combination with sweeteners, including high intensity sweeteners (such as sucralose, saccharin, aspartame, acesulfame K, brazzein, mogrosides (such as those extracted from Luo Han Guo fruit, including mogroside V) and steviol glycosides (such as those extracted from the stevia plant, including rebaudiosides and stevioside, or those obtained by fermentation or bioconversion), low calorie sweeteners (such as polyols, including erythritol, sorbitol, xylitol, lactitol, maltitol, mannitol, isomalt, etc.) and conventional caloric sweeteners (such as sucrose, glucose syrups, fructose syrups, dextrose, high fructose corn syrup, maltose, lactose, etc.).

Foodstuffs, in which the composition of the invention can be incorporated, are not limited and include:
- bakery products, both yeast-raised (including donuts, sweet doughs, breads, brioche etc.) or chemically-leavened (including cookies, cookie crisps, biscuits, muffins, cakes, brownies etc.);
- breakfast cereals (including extruded cereals, puffed cereals etc.);
- dairy products, for instance yogurt, yogurt drinks, dairy milk drinks, smoothies, ice cream, shakes, cottage cheese, dairy-based dressing, and dairy-based desserts (including mousse, puddings etc.) and the like;
- confectionery products, for instance hard candies, fondants, nougats, marshmallows, gelatin jelly candies or gummies or jelly beans, jellies, chocolate, licorice, chewing gum, caramels, toffees, chews, mints, tableted confections, and processed fruit snacks, chocolates, chocolate coatings, and icings (including frosting and glazes) and the like;
- snacks, including puffed, extruded or sheeted or texturized snacks, for instance chips/crisps, crackers;
- cereal bars, power bars, protein bars, meal replacement bars, granola bars, nutrition bars and the like;
- fruit-based jams and jellies (or other gelatin-containing desserts);
- fat-based fillings for sweet or savory foods;
- beverages, for instance carbonated beverages, fruit juices, concentrated juice mixes or syrups for dilution, clear-flavored waters, protein shakes, and beverage dry mixes for dissolution;
- and others: soups, syrups, sauces, dressings, edible films, coffee creamers, processed meats and fish, infant and toddler food, sport drinks and food, dietary supplements, clinical nutritional composition.

The composition of the invention can be used in feed or petfood (dry or moist) for animal consumption. For instance, it can be used as prebiotic composition to improve animal digestive health and/or to reduce calories and/or sugar content.

The composition of the invention can be used in personal care products, in particular cosmetics (for instance skin creams) and oral care products (for instance toothpaste). For instance, it can be used as personal care composition to improve the microbiome found on the skin. Or, for instance, it can be used in toothpaste or similar oral care compositions to improve the microbiome found in the mouth.

The composition of the invention may be utilized as a prebiotic and may also be coupled with a probiotic delivery system, for instance used in combination with one or more probiotics in a foodstuff. By "prebiotic" it is meant a food ingredient that beneficially affects the subject (human or animal) by selectively stimulating the growth and/or activity of one or a limited number of bacteria in the gastro-intestinal tract, particularly the colon, and thus improves the digestive health of the host. By "probiotic" it is meant living microbiological dietary supplements that provide beneficial effects to the subject through their passage and function in the digestive tract.

The composition of the invention may be utilized as a prebiotic and may also be coupled with a postbiotic delivery system, for instance used in combination with one or more postbiotics in a foodstuff. By "postbiotic" (also known as metabiotics, biogenics, or simply metabolites) it is meant soluble factors (metabolic products or byproducts), secreted by live bacteria, or released after bacterial lysis providing physiological benefits to the host. These are typically bioactive compounds the probiotic bacteria produce when they consume prebiotics (such as dietary fiber).

Foodstuffs of the present invention can also be used to help control the blood glucose concentration (glycemia) in humans and animals, for instance that suffer from insulin resistance, pre-diabetes or diabetes. When the foodstuff is consumed, the slowly digestible and/or digestion resistant components in the foodstuff coming from the composition of the invention can cause a more moderate relative glycemic response in the bloodstream, which can be beneficial for subjects with impaired glucose and/or insulin metabolism.

The foodstuff can also be a nutraceutical, a dietary supplement, or a functional, fortified, and/or enriched food providing health benefits to the consumer.

### DESCRIPTION OF THE DRAWINGS

Figure 1 provides an overview of the coupled reaction using maltose phosphorylase (MP) and kojibiose phosphorylase (KP).

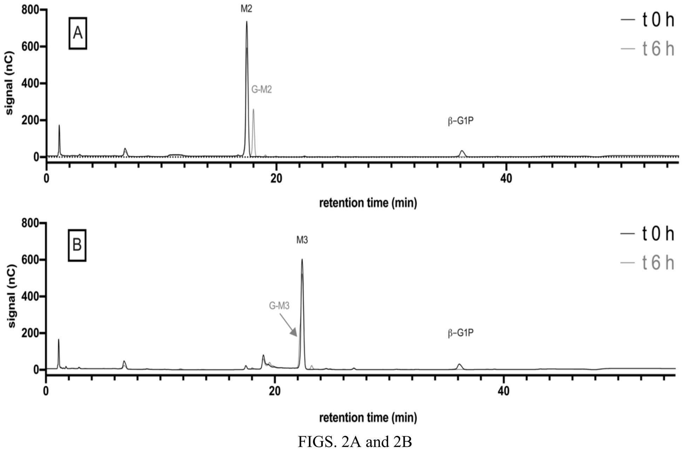

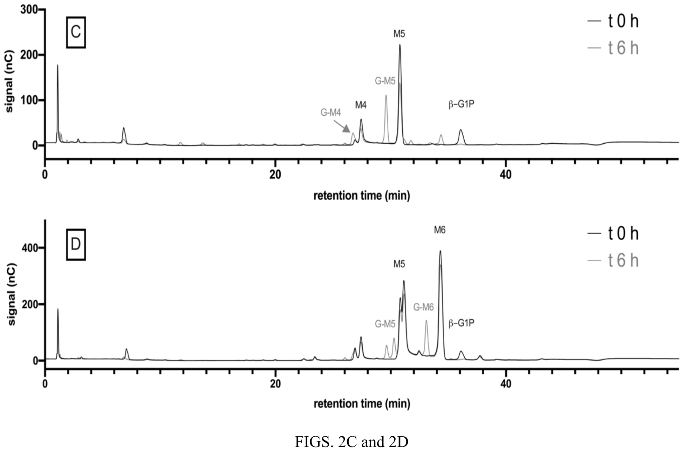

Figures 2A, 2B, 2C and 2D show: HPAEC chromatograms of the reactions of CsKP with pure gluco-oligosaccharides run with HPAEC program 1. All reactions started from 200 mM acceptor substrate and 100 mM β-G1P as donor substrate. From top to bottom acceptor substrates are: A - maltose (M2), B - maltotriose (M3), C - maltotetraose (M4) and maltopentaose (M5) and D - maltopentaose (M5) and maltohexaose (M6). Substrates are indicated in black (M2 to M6, β-G1P; t = 0 hours), glucosylated products are indicated in grey (G-M2 to G-M6; t = 6 hours).

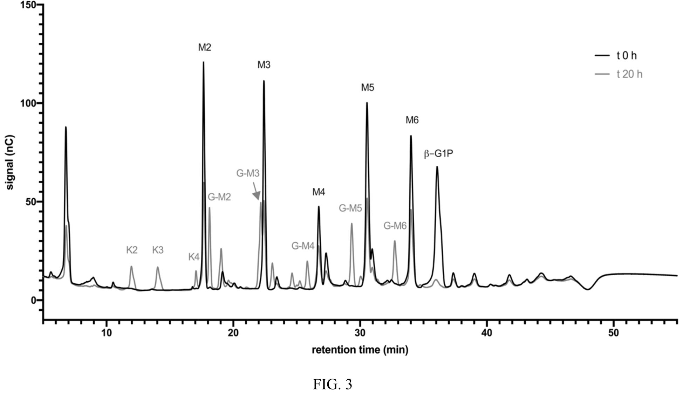

Figure 3 shows a HPAEC chromatogram of the uncoupled reaction of CsKP with a mixture of gluco-oligosaccharides, run with HPAEC program 1. The reaction started from 50 g/L commercial glucose syrup (30DE, Cargill) as acceptor substrate and 200 mM β-G1P as donor substrate, in the presence of 0.1 U/mL CsKP. The initial substrate composition is shown as the black line (t = 0 hours), with M2-M6 and β-G1P indicated in black. The resulting product mixture after a 20-hour reaction is shown as the grey line (t = 20 hours), the glucosylated products indicated in grey (G-M2 to G-M6). Beside the gluco-oligosaccharide products, the formation of kojioligosaccharides (K2-K4) in the reaction mixture is also detected.

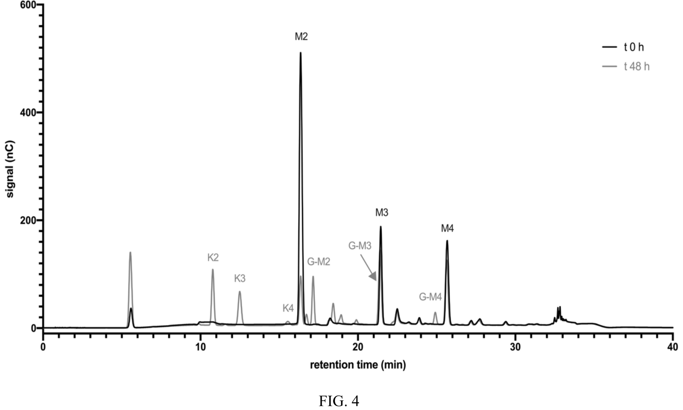

Figure 4 shows a HPAEC chromatogram of the coupled reaction of CsKP and LaMP with a mixture of gluco-oligosaccharides as substrate run with HPAEC program 2. The reaction started from 150 g/L commercial glucose syrup high in M2, M3 and M4, and 25 mM inorganic phosphate as substrates, in the presence of CsKP and LaMP. The initial substrate composition is shown as the black line (t = 0 hours), with M2-M4 indicated in black. The resulting product mixture after a 48-hour reaction is shown as the grey line, the glucosylated products indicated in grey (G-M2 to G-M4). Besides the gluco-oligosaccharide products, the formation of kojioligosaccharides (K2-K4) in the reaction mixture is also detected.

### EXAMPLES

### Enzyme production (CsKP, LaMP)

### Gene cloning and transformation

The gene encoding the C. saccharolyticus kojibiose phosphorylase (CsKP, UniProt identifier A4XGP2) was codon optimized for E. coli and synthesized. The L. acidophilus maltose phosphorylase (LaMP, UniProt identifier Q5FI04) was codon optimized for E. coli and synthesized (Life Technologies, Merelbeke, Belgium). Sequences were subsequently subcloned into a pET21 vector at NheI and XhoI restriction sites, consequently introducing a C-terminal His6-tag. The plasmid was transformed in E. coli BL21(DE3) electrocompetent cells. DNA sequences of the resulting C. saccharolyticus kojibiose phosphorylase gene (SEQ ID NO: 1) and the L. acidophilus maltose phosphorylase gene (SEQ ID NO: 3) were expressed into the respective enzymes C. saccharolyticus kojibiose phosphorylase (SEQ ID NO: 2) and L. acidophilus maltose phosphorylase (SEQ ID NO: 4) and recovered.

### Enzyme expression and recovery

An overnight culture was inoculated (2%) in 500 mL LB-Lennox medium containing 100 µg/mL ampicillin in a 2-L shake flask and incubated at 37 °C with continuous shaking at 200 rpm. The cultures were grown to OD600 0.6, and expression of both enzymes (in pET21a) was induced by adding isopropyl β-D-1-thio-galactopyranoside to a final concentration of 0.1 mM. Gene expression of CsKP took place for 16 hours at 30 °C. Gene expression of LaMP took place for 16 hours at 37 °C. The cultures were then centrifuged (15 min, 9000 rpm), and the cell pellets were frozen at - 20 °C for at least 4 hours.

To extract the enzymes, the cell pellets were thawed and dissolved in 10 mL lysis buffer consisting of 0.1 mM phenylmethylsulfonyl fluoride (PMSF), 1 mg/mL lysozyme, 10 mM imidazole and 50 mM phosphate buffered saline (PBS), pH 7.4. This suspension was incubated on ice for 30 min and sonicated three times for 3 min (Branson Sonifier 250, level 3, 50% duty cycle).

The cell debris was removed by centrifugation at 9000 rpm for 1 hour at 4 °C. The resulting cell extract was further purified by means of heat treatment. After an incubation of 1 hour at 60 °C, the heat-treated protein solutions were centrifuged two times for 30 min at 9000 rpm and the clear enzyme solution was filtered over a sterile 0.22 µm ø PES membrane filter (Millex^{®} Syringe Filters 0.22 micrometer polyethersulfone, Merck-Millipore) to remove any suspended solids and stored at 4 °C.

The resulting cell extract was further purified by nickel-nitrilotriacetic acid (Ni-NTA) chromatography as described by the supplier (MCLab, San Francisco, USA), after which the buffer was exchanged to 50 mM 2-morpholinoethanesulfonic acid (MES, pH 6.5) in a 30-kDa Amicon Ultra centrifugal filter (Merck-Millipore, Burlington, Massachusetts, USA). Protein concentration could be measured with a Nanodrop ND-1000 (Thermo Scientific, Rockford, USA) using the molecular weight and extinction coefficients calculated with the ProtParam tool on the ExPASy server (http://web.expasy.org/protparam/). Molecular weight and purity were verified by sodium dodecyl sulfate-polyacrylamide gel electrophoresis (SDS-PAGE; 10% gel).

### Determination of enzyme activity

Activity of the purified enzymes was routinely determined by phosphorolysis of kojibiose and maltose for kojibiose phosphorylase (CsKP) and maltose phosphorylase (LaMP), respectively. To enable quantification of the enzyme activity, a standard curve was made in the range of 0-500 microM glucose. The glucose standard curve and glucose released by phosphorolysis of kojibiose and maltose was quantified with a colorimetric enzymatic coupled assay using glucose oxidase and peroxidase (GOD-POD). Glucose Oxidase (GOD) converts glucose into gluconolacton, producing hydrogen peroxide. The peroxide is subsequently reduced by peroxidase (POD), using ABTS (2,2'-azino-bis(3-ethylbenzothiazoline-6-sulphonic acid)) as electron donor. The oxidized ABTSH has a green color, which can be measured at 420 nm.

Activity measurements were performed on 1-mL scale with a reaction mixture containing 100 mM kojibiose or maltose in 50 mM sodium phosphate (NaH2PO4/Na2HPO4) buffer, pH 6.5 at 55 *°*C and shaken at 800 rpm. 25-microL samples were taken at set timepoints (0, 1, 2, 4, 6, 8 and 10 min), and inactivated in 25 microL 0.2 M NaOAc buffer pH 2.5. Next, 200 microL GOD-POD reagent (173 microL POD solution (40 mg/mL in 0.2 M acetate buffer pH 4.5), 50 mg ABTS, 45.26 mg GOD, 0.2 M acetate buffer pH 4.5 to 100 mL) was added, and incubated at 37 °C for 30 min. Finally, absorbance was measured with a spectrophotometer at 420 nm. Enzyme activity (Units) was calculated as the rate of glucose release per unit of time, in micromol glucose per second (1 U = 1 micromol/s).

### Example 1 - Elongation of gluco-oligosaccharides by kojibiose phosphorylase

### Uncoupled reactions

In initial experiments the evaluation of several pure gluco-oligosaccharides as acceptor substrates for CsKP took place. Reactions with pure gluco-oligosaccharides as acceptor substrates are used to evaluate which products KP makes through glucosylation of these compounds. These reaction products could then be compared to the results achieved from a reaction starting with a mixture of gluco-oligosaccharides as substrate for KP.

Reactions were performed on a 1-mL scale, starting from 200 mM of the respective acceptor substrate, 100 mM β-D-glucose-1-phosphoric acid (β-G1P), and 0.5 U/mL CsKP in 50 mM 2-(N-morpholino)ethanesulfonic acid (MES) buffer at pH 6.5, 55 °C and 800 rpm. The chosen acceptor substrates were maltose (M2), maltotriose (M3), maltotetraose (M4), maltopentaose (M5), and maltohexaose (M6). Maltose (M2) was purchased from Sigma-Aldrich, all pure gluco-oligosaccharides (M3-M6) were purchased from Carbosynth in the highest purity available, and β-G1P was produced in-house (Van Der Borght, Desmet and Soetaert, 2010).

Samples (50 microL) were taken at the beginning (t = 0 hours) and at the end of the reaction (t = 20 hours), inactivated in 100 mM NaOH and further diluted to a 200-fold dilution for high performance anion exchange chromatography (HPAEC) analysis.

All HPAEC analysis with integrated pulsed amperometric detection (HPAEC-IPAD) were performed on a CarboPac PA20 3x150mm column, using the ICS-6000 Dionex system (Thermo Fisher Scientific) and a flow rate of 0.5 mL/min. In order to separate the respective carbohydrates, two protocols were used. **HPAEC program 1** is a 55-min protocol. During the first 4 min, an isocratic elution with 80 % eluent A (ddH20) and 20 % eluent B (100 mM NaOH) was used. Between 4 min and 8 min, eluent B was linearly increased from 20 % to 100 %, and maintained at 100 % until 10 min. Between 10 min and 45 min, eluent C (1 M NaOAc and 100 mM NaOH) was increased linearly from 0 % to 15 %. Next, between 45 min and 46 min, eluent C and B were decreased linearly to 0 % and 20 %, respectively. Thereby, the initial isocratic elution with 80 % eluent A and 20 % eluent B was restored and maintained until 55 min. If needed, standards of 20 µM were run to enable detection of the known compounds.

**HPAEC program 2** is a 40-min protocol. During the first 4 min, an isocratic elution with 80 % eluent A (ddH20) and 20 % eluent B (100 mM NaOH) was used. Between 4 min and 8 min, eluent B was linearly increased from 20 % to 100 %, and maintained at 100 % until 10 min. Between 10 min and 30 min, eluent C (1 M NaOAc and 100 mM NaOH) was increased linearly from 0 % to 10 %. Between 30 min and 30.5 min, eluent C was linearly increased from 10 % to 30 %, and maintained at 30 % until 33 min. Next, between 33 min and 33.5 min, eluent C and B were decreased linearly to 0 % and 20 %, respectively. Thereby, the initial isocratic elution with 80 % eluent A and 20 % eluent B was restored and maintained until 40 min. If needed, standards of 20 µM were run to enable detection of the known compounds.

From the initial reactions it became clear that CsKP is capable of utilizing gluco-oligosaccharides with a DP (the degree of polymerization) of 2-6 as acceptor substrate, resulting in the formation of glucosylated products (Figures 2A-2D). In Figure 2A, the glucosylated product (G-M2) appears to the right of the substrate (M2) on the HPAEC chromatogram. For maltotriose (M3), shown in Figure 2B, the glucosylated product (G-M3) appears only slightly to the left, showing overlap with the substrate. The products (G-M4, G-M5, G-M6) of the larger substrates, maltotetraose (M4), maltopentaose (M5), and maltohexaose (M6), all appear clearly to the left of the respective substrates, as indicated in Figures 2C and 2D.

A reaction starting from 50 g/L commercial glucose syrup (30DE, Cargill) as acceptor substrate and 200 mM β-G1P as donor substrate was evaluated. The reaction was performed on a 1-mL scale at 55 °C, 50 mM MES buffer pH 6.5, 800 rpm, and 0.1 U/mL CsKP was added. 50-microL samples were taken at certain timepoints, inactivated in an equal part of 100 mM NaOH, centrifuged at 13 000 rpm for 10 min, and further diluted to a 200-fold dilution for HPAEC analysis following the abovementioned protocol.

The reaction starting from a commercial glucose syrup (30DE, Cargill) shows that CsKP is capable of utilizing gluco-oligosaccharides with a DP of 2-6 as acceptor substrate, resulting in the formation of a mixture of glucosylated products (Figure 3). These products clearly resemble the products formed in the reactions with pure gluco-oligosaccharides substrate (Figures 2A-2D). Indeed, the glucosylated product (G-M2) again appears to the right of the substrate (M2) on the HPAEC chromatogram (Figure 3). For maltotriose (M3), the glucosylated product (G-M3) appears only slightly to the left, showing overlap with the substrate. The products (G-M4, G-M5, G-M6) of the larger substrates, maltotetraose (M4), maltopentaose (M5), and maltohexaose (M6), all appear clearly to the left of the respective substrates. Besides the gluco-oligosaccharide products, the formation of kojioligosaccharides (K2-K4) in the reaction mixture is also detected. This is an intrinsic result of the presence of glucose in the reaction mixture, which CsKP uses as an acceptor substrate to form kojioligosaccharides.

Surprisingly, all products appeared to be formed in similar amounts, with respect to the relative initial amount of the corresponding gluco-oligosaccharides. This is shown in Table 1 showing the area of each HPAEC peak for each product.

**Table 1.**

| Peak area (nC*min) | **DP2¹** | **DP3** | **DP4** | **DP5** | **DP6** |
|---|---|---|---|---|---|
| **Before reaction Mx²** | 11.25 | 10.70 | 6.81 | 14.01 | 12.82 |

| | [M2] | [M3] | [M4] | [M5] | [M6] |
|---|---|---|---|---|---|
| **After reaction elongated oligosaccharide G-Mx** | 7.60 [G-M2] | 9.59 [G-M3] | 3.50 [G-M4] | 9.20 [G-M5] | 7.23 [G-M6] |

| | | | | | |
|---|---|---|---|---|---|
| ¹ DP = Degree of polymerization. ² Mx: x is respective degree of polymerization. | | | | | |

Thus, CsKP appears to be able to glucosylate gluco-oligosaccharides of different chain length in a mixture with a comparable efficiency for substrates ranging from DP2 to DP6. This finding is particularly interesting, as it indicates that cheap and readily available commercial maltodextrin mixtures or glucose syrups can be used as a substrate to be elongated by kojibiose phosphorylase, with no significant preference for a certain DP in the range of 2 to 6. This eliminates the need to start from costly, highly pure gluco-oliogosaccharides with a defined DP, as a comparable conversion of all gluco-oligosaccharides s will be achieved, even when present in a mixture.

### Coupled reaction

In a next step, the possibility of the *in situ* production of β-G1P by maltose phosphorylase from *Lactobacillus acidophilus* (LaMP) was evaluated. This implies the employment of a coupled bi-enzymatic process, comprising both MP and KP. The first enzymatic process uses inorganic phosphate to perform phosphorolysis of the maltose present in the reaction mixture, leading to the production of β-G1P (Hüwel *et al.,* 1997). Next, in the second enzymatic process, KP can use the released β-G1P as a donor substrate to elongate the gluco-oligosaccharides present in the reaction mixture.

In that respect, a reaction starting from 150 g/L gluco-oligosaccharide mixture high in M2, M3 and M4 as substrate was evaluated. The reaction was performed on a 1-mL scale at 55 °C, 50 mM MES buffer pH 6.5, 800 rpm, and 0.2 U/mL LaMP and 0.1 U/mL CsKP were added. 50-micro samples were taken at certain timepoints, inactivated in an equal part of 100 mM NaOH, centrifuged at 13 000 rpm for 10 min, and further diluted to a 200-fold dilution for HPAEC analysis following the abovementioned protocol.

The coupled reaction shows that CsKP is capable of elongating gluco-oligosaccharides with a DP of 2-4 (M2, M3, M4) in a coupled reaction, resulting in the formation of a mixture of glucosylated products (Figure 4). These products clearly resemble the products formed in the reactions with pure gluco-oligosaccharides (Figures 2A-2D), and the products formed in the single-enzyme process (Figure 3). Indeed, the glucosylated product of maltose (G-M2) again appears to the right of the substrate (M2) on the HPAEC chromatogram (Figure 4). For maltotriose (M3), the glucosylated product (G-M3) appears only slightly to the left, showing overlap with the substrate. The product of maltotetraose (G-M4) appears clearly to the left of its respective substrate (M4). In a similar fashion to the previous reactions (Figure 3), also here the formation of kojioligosaccharides (K2-K4) in the reaction mixture is detected.

### Example 2 - Elongation of other malto-oligosaccharide compositions by kojibiose phosphorylase Coupled reaction

Reactions starting from 200 g/L gluco-oligosaccharide mixtures as acceptor composition were evaluated; either maltose syrup or mixtures of DP3 syrup with maltose. Each reaction was performed on a 1-mL scale at 55 °C, 50 mM MES buffer pH 6.5, 800 rpm, and 0.4 U LaMP per mL reaction mixture and 0.1 U/mL CsKP were added. Additionally, 0.4 U LaMP /mL reaction mixture was added every 24 hours. The experiments were carried out with or without a glucose-isomerase (GI) added (see Table 2), being an immobilized glucose isomerase, Sweetzyme IT extra (Novozymes) of which 70 U per mL reaction mixture was added. 50-microL samples were taken at certain timepoints, inactivated in an equal part of 100 mM NaOH, centrifuged at 13 000 rpm for 10 min, and further diluted to a 200-fold dilution for HPAEC analysis following the abovementioned protocol 1. For some incubations (Table 2, #5 and #6), part of the maltose substrate was added at the beginning of the incubation, while the remaining part was added after 24 hours of reaction. Total reaction time was 72 hours.

Immobilized glucose isomerase was used in experiments #1, #3 and #6 to obtain syrups that are lower in glucose content, having part of the glucose converted into fructose (ratio glucose/fructose of around 58/42). In this way, fructose containing syrups were prepared that gave a lower glycemic index as compared to the original syrups.

The HPAEC chromatograms obtained at the end of the reactions showed peaks for the following glucosylated products for the experiments #1, #2, #4-#6: G-M2, K3 + K4. The amount of G-M2 found in #1 did not differ from the amount in #2, the isomerization process had no influence on the amount of G-M2. When comparing #3 and #4 a decrease in G-M2 with a factor 1.2 was noted, going from #4 to #3. When comparing #5 and #6, a decrease in G-M2 with a factor 2.4 was noted, going from #6 to #5.

When comparing #3 and #5 it was noted that the amount of maltose is 3 times the amount of M3 in #3 right from the start. For #5, extra maltose was added after 24 hours from the start of the incubation to a final ratio of 3 times the amount of M3 (the same ratio as for #3). The effect from this later M2 addition, is that when going from #3 to #5, the G-M2 is decreased with a factor 1.4.

**Table 2.**

| # | Polypeptides | Acceptor composition |
|---|---|---|
| 1. | LaMP + CsKP + GI | Maltose syrup¹ |
| 2. | LaMP + CsKP | Maltose syrup¹ |
| 3. | LaMP + CsKP + GI | DP3²/M2³ (1:3 w/w) |
| 4. | LaMP + CsKP | DP3²/M2³ (1:3 w/w) |
| 5. | LaMP + CsKP + GI | DP3²/M2³ (1:1 w/w) and M2 till 1:3 after 24 hours |
| 6. | LaMP + CsKP | DP3²/M2³ (1:1 w/w) and M2 till 1:3 after 24 hours |

| | | |
|---|---|---|
| ¹ Maltose syrup containing 82 wt.% of maltose (M2). ² DP3 syrup having 95.8 wt.% of maltotriose (M3). ³ M2 is pure maltose. | | |

### Example 3 - Elongation of other malto-oligosaccharide compositions by kojibiose phosphorylase Coupled reaction

Reactions starting from a total of 200 g/L gluco-oligosaccharide mixtures as substrate were evaluated. Each reaction was performed on a 1-mL scale at 55 °C, 10 mM sodium phosphate buffer pH 6.5, 800 rpm, and 0.4 U LaMP per mL reaction mixture [at 0, 24, 48, and 72 hours 0.4 U/mL was added] and 0.8 U CsKP per ml reaction mixture were added. 500-microL samples were taken at certain timepoints, inactivated by heating at 99 °C for 10 minutes, and further diluted to 20 mg/L and treated with mixed bed ion exchange resin (AG501-X8 (D), Bio-Rad). to desalt, and filtered over a 0.45 micron disposable filter, for HPAEC analysis following the abovementioned protocol 1. Total reaction time was 96 hours. The HPAEC chromatograms at the end of the reactions gave again the characteristic peaks of the elongated oligosaccharides as seen in Example 1.

The starting syrups that were used for the elongation experiments are shown in Table 3. Each starting syrup was mixed with maltose in the following ratios (syrup to maltose, w/w, ds): 1 to 0 (no mixing, pure syrup); 1 to 0.1; 1 to 0.25; 1 to 0.5 to a final substrate concentration of 200g/L.

**Table 3.**

| Starting Syrup | 1 | 2 | 3 |
|---|---|---|---|
| Composition in wt.% | | | |
| DP1 | 2.6 | 3.2 | 3 |
| DP2 | 11.9 | 14.3 | 13.1 |
| DP3 | 13.9 | 18.7 | 15.5 |
| DP4 | 6.5 | 9.4 | 7.6 |
| DP5 | 17.3 | 14.9 | 19.3 |
| DP6 | 17 | 23.7 | 21.4 |
| DP7 | 2.2 | 6.8 | 3.5 |
| DP8 | 2 | 1.1 | 1.6 |
| DP9 | 1.9 | 0.8 | 1.3 |
| DP10 | 1.6 | 0.7 | 0.7 |
| DPn | 23.1 | 6.4 | 13 |

In total 12 syrups were prepared:
1. Starting syrup 1
   a. Pure starting syrup 1
   b. 1 part starting syrup 1 and 0.1 part maltose
   c. 1 part starting syrup 1 and 0.25 part maltose
   d. 1 part starting syrup 1 and 0.5 part maltose
2. Starting syrup 2
   a. Pure starting syrup 2
   b. 1 part starting syrup 2 and 0.1 part maltose
   c. 1 part starting syrup 2 and 0.25 part maltose
   d. 1 part starting syrup 2 and 0.5 part maltose
3. Starting syrup 3
   a. Pure starting syrup 3
   b. 1 part starting syrup 3 and 0.1 part maltose
   c. 1 part starting syrup 3 and 0.25 part maltose
   d. 1 part starting syrup 3 and 0.5 part maltose

The twelve different syrup combinations (1a-d, 2a-d, and 3a-d) were each analyzed for the production of G-M2, as a representative member of alpha-(1,2)-elongated gluco-oligosaccharides. For the different syrups it was noticed that the alpha-(1,2)-elongated gluco-oligosaccharides of different degrees of polymerization were also produced, similar to discussed above in Example 1.

When comparing 1a with 1b, an increase in G-M2 with a factor 1.6 was observed for 1b. Further increase of the initial maltose content (1c) gave an increase of G-M2 with a factor 3.1, whereas 1d gave an increase with a factor 4.8. A similar increase in G-M2 was observed when syrup 2 was enriched with maltose. When going from 2a to 2b, an increase in G-M2 with a factor 2.3 was observed. When going from 2a to 2c an increase of G-M2 with a factor 4.0 was found and when going from 2a to 2c an increase with a factor 6.3 was found. A similar increase in G-M2 was observed when syrup 3 was enriched with maltose. When going from 3a to 3b, an increase in G-M2 with a factor 2.3 was observed. When going from 3a to 3c an increase of G-M2 with a factor 4.0 was found and when going from 3a to 3c an increase with a factor 7.3 was found.

For all starting syrups (1, 2 and 3), the formation of G-M2 after enzymatic reaction increased with increasing initial maltose content mixed with these.

### Example 4 - non-digestibility of elongated oligosaccharides

### Production of the elongated syrup

A 500-mL scale reaction was performed in a 1-L Erlenmeyer starting with the following reaction conditions: 20 % ds Very High Maltose Syrup (Cargill, 1,2% DP1, 78% DP2, 19% DP3), 10 mM sodium phosphate buffer pH 6.5, 0.4 U/mL LaMP and 0.8 U/mL CsKP in a 50 mM 2-(N-morpholino)ethanesulfonic acid (MES) buffer at pH 6.5, 55 °C and 200 rpm. The reaction proceeded for 144 hours in total (= 6 days). Additionally, 0.4 U LaMP /mL reaction mixture was added every 24 hours. Samples (50 microL) were taken at the beginning of the reaction and every 24 hours before addition of LaMP and at the end of the reaction (t = 144 hours), inactivated in a 100 °C heat block for 10 min and further diluted to a 400-fold dilution for HPAEC analysis according to protocol 1 discussed above.

The 500-mL reaction was terminated through heat-inactivation of the enzymes by heating in a water bath at 100 °C for 10 min. Next, the reaction mixture was cooled to 4 °C for further purification. For the purification, the carbohydrate reaction mixture was first filtered with a Whatman filter (Silicone treated, circles, 90 mm) to eliminate the precipitated proteins. Next, a 0.2 micron PES-filter unit (250 mL volume, VWR Vacuum filtration) was used to eliminate the smallest particles and obtain a highly transparent, light yellow solution.

To eliminate all unwanted ions and color bodies the reaction mixture was treated with a mixed bed ion exchange resin (AG501-X8 (D), Bio-Rad). Finally, the resin was removed by vacuum filtration with a 0.2 micrometer PES-filter. A highly transparent, colorless mixture with a brix of 20 was obtained.

In a last step, the obtained product mixture was evaporated in a rotavapor to obtain a highly viscous and microbiologically stable syrup with a brix of 80. HPAEC analysis according to protocol 1 discussed above showed the characteristic signals for α-1,2-elongated oligosaccharides. The composition of the α-1,2-elongated oligosaccharide syrup is depicted in Table 4.

**Table 4.**

| Component | w/w % on d.s. |
|---|---|
| Glucose (glc) | 10.4 |
| Maltose (M2) | 9.5 |
| Maltotriose (M3) | 13.2 |
| Kojibiose (K2) | 9.6 |
| Kojitriose (K3) | 8.6 |
| Kojitetraose (K4) | 3.0 |
| Kojipentose (K5) | 1.8 |
| Iso-Kojitriose ((i)DP3) | 28.8 |
| 2 or 3 glucose units via alpha(1,2) linkages on maltose (K2/3-M2) | 5.9 |
| Iso-Kojitetraose ((i)DP4) | 8.4 |
| 2 glucose units via alpha(1,2) linkages on (iso)maltotriose or iso-Kojipentose (K2-(i)M3 or (i)DP5) | 0.9 |

### Analysis of the elongated syrup

For in vitro digestion analysis, the method described in Garcia-Campayo, *et al.* was used. (Garcia-Campayo et al., "Digestion of food ingredients and food using an in vitro model integrating intestinal mucosal enzymes," Food and Nutrition Sciences, 2018, 9: 711-734). Native corn starch (Cargill 03420) was used as a reference for digestible carbohydrate. Moisture content was determined by HR73 Halogen Moisture Analyzer at 130°C in duplicate.

Briefly, in vitro digestion was performed in three sequential phases: saliva phase (5 min), gastric phase (2 hours), and small intestinal phase (4 hours). Each sample was independently digested in 15 mL conical in triplicate (n=3). For each digestion tube, the amount of sample added per digestion was normalized to deliver 30 mg dry solids. The native corn starch was added directly to the digestion tube. For the syrup of elongated oligosaccharides, a stock solution of 300 mg dry solids was prepared in 10 mL saliva cocktail, then 1 mL was added to each digestion tube.

Free glucose content of the digesta was determined by an enzymatic (GOPO)-colorimetric reaction using Stanbio glucose Liquicolor reagent (Stanbio Laboratories). Briefly, 10 microL of sample or glucose standard (0-4 mg/mL) were mixed with 1 mL glucose reagent and incubated at room temperature for 30 min. Absorbance was measured at 500 nm (Synergy HT, Bioteck).

Results are presented in Table 5 as % glucose released per total glucose (g glucose / 100 g total monomeric + polymeric glucose). As seen from Table 5, after 6 hours about 50 % of the elongated oligosaccharides syrup was non-digestible.

**Table 5.**

| Total time (hours) | Corn starch | Elongated oligosaccharides syrup |
|---|---|---|
| 0 | 0.0 ± 0.0 | 11.5 ± 1.0 |
| 2 | 0.0 ± 0.0 | 11.5 ± 2.0 |
| 2.5 | 15.8 ± 3.2 | 34.7 ± 3.1 |
| 3 | 38.3 ± 2.0 | 41.3 ± 0.2 |
| 4 | 66.8 ± 5.1 | 46.8 ± 1.5 |
| 5 | 74.6 ± 2.2 | 49.9 ± 1.2 |
| 6 | 90.5 ± 7.0 | 48.5 ± 0.5 |

### Conclusions

In a first stage, the ability of KP to glucosylate gluco-oligosaccharides was evaluated, and product formation was confirmed by HPAEC analysis. Next, the ability of KP to glucosylate several gluco-oligosaccharides of different DPs when present in a substrate mixture was analyzed in more detail.

The inventors succeeded for the first time in producing a product mixture containing alpha-(1,2)-elongated gluco-oligosaccharides of different DPs through the glycosylation activity of a kojibiose phosphorylase. This was both achieved in an uncoupled reaction, starting from gluco-oligosaccharides, β-glucose 1-phosphate and a kojibiose phosphorylase (Figure 3), as well as in a coupled bi-enzymatic reaction where β-glucose 1-phosphate was produced *in situ* by the activity of a maltose phosphorylase (Figure 4).

Both processes led to the elongation of at least two gluco-oligosaccharides substrates with a DP of n, wherein n is 3, 4, 5 or 6 and wherein each gluco-oligosaccharide comprises at least one alpha-(1,4) linkage, an intrinsic characteristic of partially hydrolyzed starch compositions. The alpha-(1,2)-elongation of these substrates by KP results in a product mixture of the at least two different gluco-oligosaccharides each with at least one alpha-(1,2)-linkage and at least one alpha-(1,4)-linkage, with a DP of n+1.

### REFERENCES

- Van Der Borght, J., Desmet, T. and Soetaert, W. (2010) 'Enzymatic production of β-D-glucose-1-phosphate from trehalose', Biotechnology Journal, 5, pp. 986-993. doi: 10.1002/biot.201000203.
- Hüwel, S. et al. (1997) 'Maltose phosphorylase from Lactobacillus brevis: Purification, characterization, and application in a biosensor for ortho-phosphate', Enzyme and Microbial Technology, 21(6), pp. 413-420. doi: 10.1016/S0141-0229(97)00014-8.
- Nakada, T. et al. (2003) 'Kojioligosaccharides: Application of Kojibiose Phosphorylase on the Formation of Various Kojioligosaccharides', in Oligosaccharides in Food and Agriculture, pp. 104-117.
- Yamamoto, T. et al. (2006) 'Improvement of the Enzymatic Properties of Kojibiose Phosphorylase from Thermoanaerobacter brockii by Random Mutagenesis and Chimerization', 129.
- Yamamoto, T. et al. (2011) 'Enzymatic Properties of Recombinant Kojibiose Phosphorylase from Caldicellulosiruptor saccharolyticus ATCC43494', Bioscience, Biotechnology, and Biochemistry, 75(6), pp. 1208-1210. doi: 10.1271/bbb.110116.
- Starch: Chemistry and Technology, Third Edidtion, ISBN: 978-0-12-746275-2 Chapter 21, "Sweeteners from Starch: Production, Properties and Uses"
- Garcia-Campayo et al., "Digestion of food ingredients and food using an in vitro model integrating intestinal mucosal enzymes," Food and Nutrition Sciences, 2018, 9: 711-734

### CLAUSES

1. A process for elongating gluco-oligosaccharides with at least one α-(1,2)-terminally linked glucosyl moiety, comprising the steps of:
   providing an acceptor composition comprising gluco-oligosaccharides having a degree of polymerization of 2, 3, 4, 5 or 6 glucosyl moieties, wherein each gluco-oligosaccharide comprises at least one α-(1,4)-linkage and wherein the acceptor composition comprises at least two gluco-oligosaccharides having a different degree of polymerization;
   placing the acceptor composition in fluid contact with a polypeptide having kojibiose phosphorylase activity in the presence of a 1-glucosyl phosphate donor.
2. The process according to clause 1, wherein the acceptor composition comprises at least 3, preferably at least 4 gluco-oligosaccharides having a different degree of polymerization.
3. The process according to clause 1 or 2, wherein the acceptor composition is a partially hydrolyzed starch having a dextrose equivalent (DE) of less than 60, preferably less than 50, more preferably less than 45.
4. The process according to clause 3, wherein the partially hydrolyzed starch is a maltodextrin or glucose syrup.
5. The process according to any one of clause 1 to 4, wherein the acceptor composition comprises at least two different gluco-oligosaccharides selected from the group consisting of maltose, maltotriose, maltotetraose, maltopentaose and maltohexaose.
6. The process according to any one of the preceding clauses, wherein the 1-glucosyl phosphate donor is β-D-glucose-1-phosphate.
7. The process according to any one of the preceding clauses, wherein the polypeptide having kojibiose phosphorylase activity is a recombinant kojibiose phosphorylase, preferably obtainable from a bacterium selected from the group consisting of *Thermococcus barophilus, Pseudothermotoga thermarum, Thermoanaerobacter brockii, Thermoanaerobacterium thermosacharolyticum, Caldicellulosiruptor saccharolyticus, Pyrococcus sp., Palaeococcus pacificus and Thermofilum pendens.*
8. The process according to any one of the preceding clauses, wherein the process further comprises a step of preparing the 1-glucosyl phosphate donor by placing maltose in fluid contact with a polypeptide comprising maltose phosphorylase activity, and phosphoric acid and/or a salt thereof.
9. The process according to clause 8, wherein the polypeptide comprising maltose phosphorylase activity is a recombinant maltose phosphorylase, preferably obtainable from a bacterium selected from the group consisting of *Lactobacillus acidophilus, Bacillus selenitireducens, Enterococcus faecalis, Lactobacillus brevis, Lactobacillus sanfranciscensis, Paenibacillus sp.* and *Bacillus sp.*
10. The process according to any one of clauses 1 to 7, wherein the process further comprises a step of preparing the 1-glucosyl phosphate donor by placing trehalose in fluid contact with a polypeptide comprising trehalose phosphorylase activity, and phosphoric acid and/or a salt thereof.
11. A composition comprising elongated gluco-oligosaccharides obtainable by the process according to any one of the preceding clauses.
12. A composition comprising gluco-oligosaccharides elongated with at least one α-(1,2)-terminally linked glucosyl moiety, wherein the gluco-oligosaccharides have a degree of polymerization of 2, 3, 4, 5 or 6; and wherein the composition comprises at least two gluco-oligosaccharides having a different degree of polymerization and wherein each gluco-oligosaccharide comprises at least one α-(1,4)-linkage.
13. The composition according to clause 11 or 12, wherein the elongated gluco-oligosaccharides comprise saccharide linkages selected from α-(1,2)-; α-(1,4)- and other saccharide linkages; wherein the amount of α-(1,4)-linkages is more than 50% and the amount of α-(1,2)-linkages is less than 50% of all saccharide linkages, excluding the linkages from kojibiose and kojioligosaccharides.
14. The composition according to any one of clauses 11 to 13, further containing one or more of kojibiose, kojitriose, kojitetraose and kojipentaose.
15. The composition according to any one of clauses 11 to 14, comprising at least 20 wt.% of elongated gluco-oligosaccharides based on the total weight of the product as dry substance.
16. The composition according to any of clauses 11 to 15, comprising at least two different elongated gluco-oligosaccharides selected from: 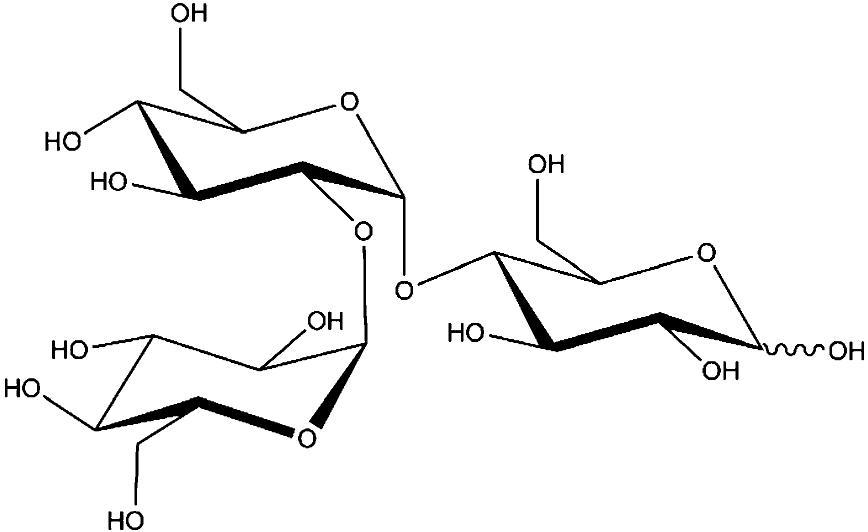 2-O-α-D-glucosyl-maltose
   (α-D-Glucopyranosyl-(1→2)-α-D-glucopyranosyl-(1→4)-D-glucopyranose) 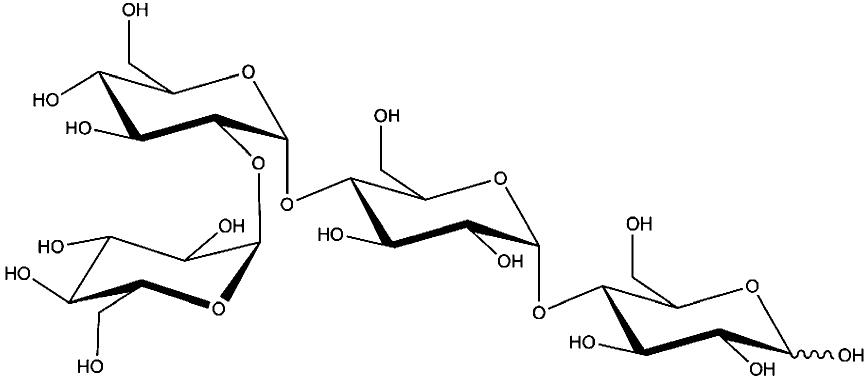 2-O-α-D-glucosyl-maltotriose
   (α-D-Glucopyranosyl-(1→2)-α-D-glucopyranosyl-(1→4)-α-D-glucopyranosyl-(1→4)-D-glucopyranose) 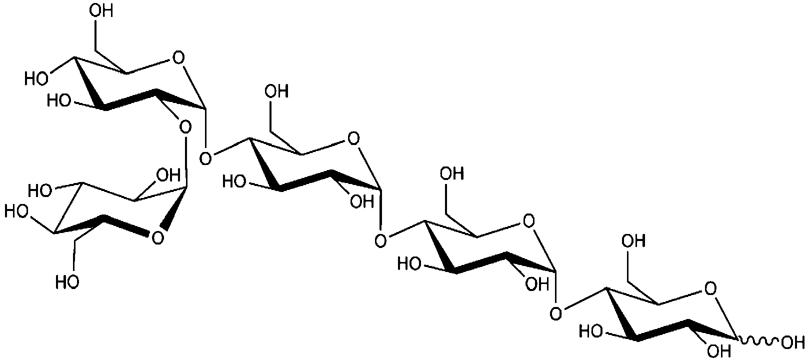 2-O-α-D-glucosyl-maltotetraose
   (α-D-Glucopyranosyl-(1→2)-α-D-glucopyranosyl-(1→4)-α-D-glucopyranosyl-(1→4)-α-D-glucopyranosyl-(1→4)-D-glucopyranose) 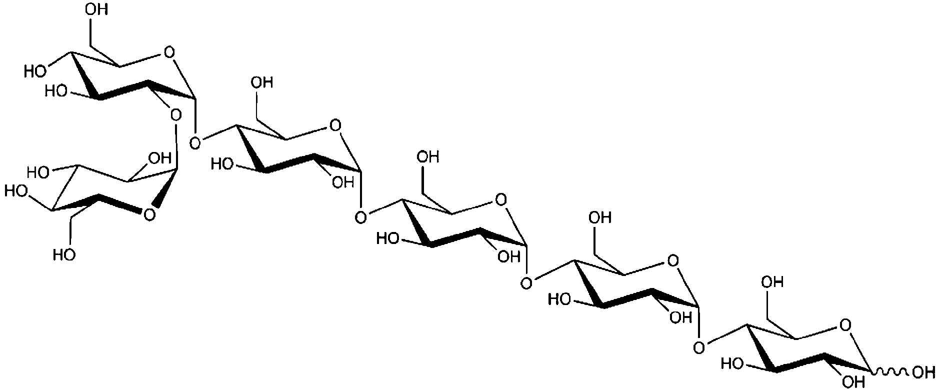 2-O-α-D-glucosyl-maltopentaose
   (α-D-Glucopyranosyl-(1→2)-α-D-glucopyranosyl-(1→4)-α-D-glucopyranosyl-(1→4)-α-D-glucopyranosyl-(1→4)-α-D-glucopyranosyl-(1→4)-D-glucopyranose) 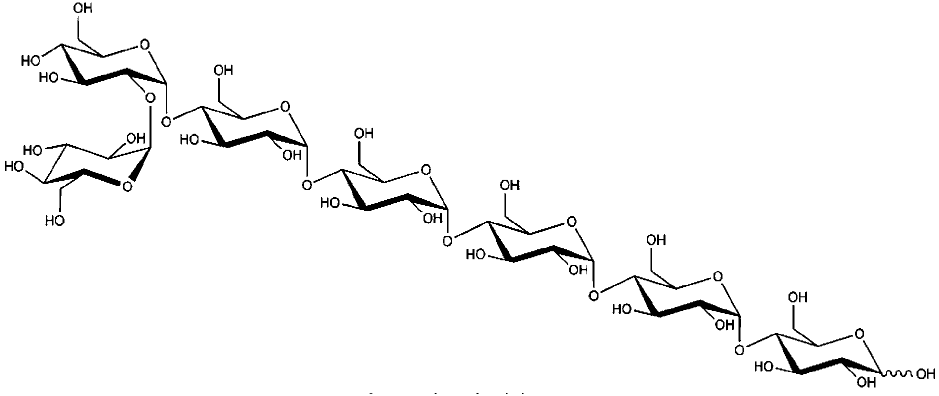 2-O-α-D-glucosyl-maltohexaose
   (α-D-Glucopyranosyl-(1→2)-α-D-glucopyranosyl-(1→4)-α-D-glucopyranosyl-(1→4)-α-D-glucopyranosyl-(1→4)-α-D-glucopyranosyl-(1→4)-α-D-glucopyranosyl-(1→4)-D-glucopyranose)
17. A foodstuff, pet food, feed or personal care product comprising the composition according to any one of clauses 11 to 16.

## Claims

1. A composition comprising elongated gluco-oligosaccharides obtainable by a process for elongating gluco-oligosaccharides with at least one α-(1,2)-terminally linked glucosyl moiety, comprising the steps of:
providing an acceptor composition comprising gluco-oligosaccharides having a degree of polymerization of 2, 3, 4, 5 or 6 glucosyl moieties, wherein each gluco-oligosaccharide comprises at least one α-(1,4)-linkage and wherein the acceptor composition comprises at least two gluco-oligosaccharides having a different degree of polymerization;
placing the acceptor composition in fluid contact with a polypeptide having kojibiose phosphorylase activity in the presence of a 1-glucosyl phosphate donor,
the composition further comprising one or more of kojibiose, kojitriose, kojitetraose and kojipentaose.

2. The composition according to claim 1, wherein the acceptor composition comprises at least 3, preferably at least 4 gluco-oligosaccharides having a different degree of polymerization.

3. The composition according to claim 1 or 2, wherein the acceptor composition is a partially hydrolyzed starch having a dextrose equivalent (DE) of less than 60, preferably less than 50, more preferably less than 45.

4. The composition according to claim 3, wherein the partially hydrolyzed starch is a maltodextrin or glucose syrup.

5. The composition according to any one of claims 1 to 4, wherein the acceptor composition comprises at least two different gluco-oligosaccharides selected from the group consisting of maltose, maltotriose, maltotetraose, maltopentaose and maltohexaose.

6. The composition according to any one of the preceding claims, wherein the 1-glucosyl phosphate donor is β-D-glucose-1-phosphate.

7. The composition according to any one of the preceding claims, wherein the polypeptide having kojibiose phosphorylase activity is a recombinant kojibiose phosphorylase, preferably obtainable from a bacterium selected from the group consisting of *Thermococcus barophilus, Pseudothermotoga thermarum, Thermoanaerobacter brockii, Thermoanaerobacterium thermosacharolyticum, Caldicellulosiruptor saccharolyticus, Pyrococcus sp., Palaeococcus pacificus and Thermofilum pendens.*

8. The composition according to any one of the preceding claims, wherein the process further comprises a step of preparing the 1-glucosyl phosphate donor by placing maltose in fluid contact with a polypeptide comprising maltose phosphorylase activity, and phosphoric acid and/or a salt thereof.

9. The composition according to claim 8, wherein the polypeptide comprising maltose phosphorylase activity is a recombinant maltose phosphorylase, preferably obtainable from a bacterium selected from the group consisting of *Lactobacillus acidophilus, Bacillus selenitireducens, Enterococcus faecalis, Lactobacillus brevis, Lactobacillus sanfranciscensis, Paenibacillus sp.* and *Bacillus sp.*

10. The composition according to any one of claims 1 to 7, wherein the process further comprises a step of preparing the 1-glucosyl phosphate donor by placing trehalose in fluid contact with a polypeptide comprising trehalose phosphorylase activity, and phosphoric acid and/or a salt thereof.

11. A composition comprising gluco-oligosaccharides elongated with at least one α-(1,2)-terminally linked glucosyl moiety, wherein the gluco-oligosaccharides have a degree of polymerization of 2, 3, 4, 5 or 6; and wherein the composition comprises at least two gluco-oligosaccharides having a different degree of polymerization and wherein each gluco-oligosaccharide comprises at least one α-(1,4)-linkage,
the composition further comprising one or more of kojibiose, kojitriose, kojitetraose and kojipentaose.

12. The composition according to any one of claims 1 to 11, wherein the elongated gluco-oligosaccharides comprise saccharide linkages selected from α-(1,2)-; α-(1,4)- and other saccharide linkages; wherein the amount of α-(1,4)-linkages is more than 50% and the amount of α-(1,2)-linkages is less than 50% of all saccharide linkages, excluding the linkages from kojibiose and kojioligosaccharides.

13. The composition according to any one of claims 1 to 12, comprising at least 20 wt.% of elongated gluco-oligosaccharides based on the total weight of the product as dry substance.

14. The composition according to any of claims 11 to 13, comprising at least two different elongated gluco-oligosaccharides selected from: 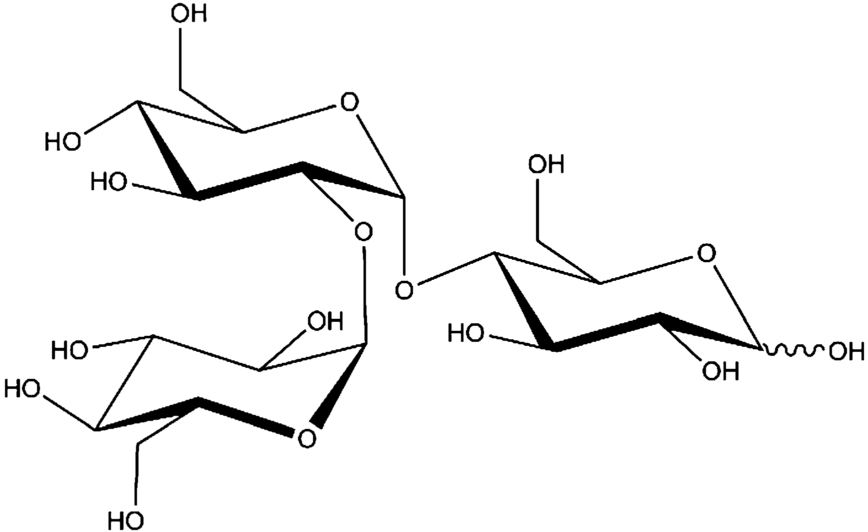 2-O-α-D-glucosyl-maltose
(α-D-Glucopyranosyl-(1→2)-α-D-glucopyranosyl-(1→4)-D-glucopyranose) 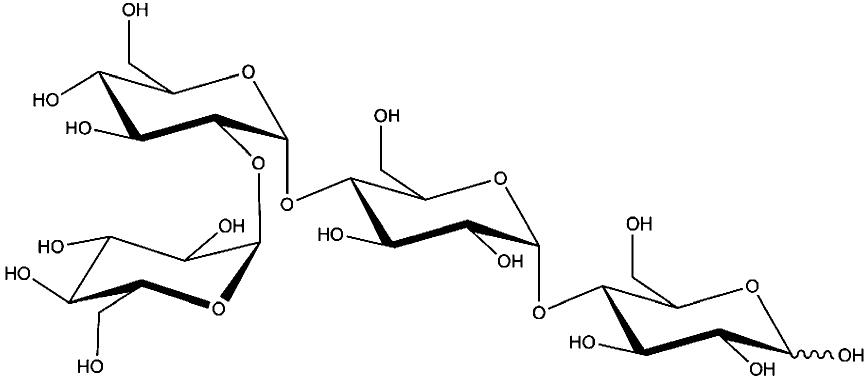 2-O-α-D-glucosyl-maltotriose
(α-D-Glucopyranosyl-(1→2)-α-D-glucopyranosyl-(1→4)-α-D-glucopyranosyl-(1→4)-D-glucopyranose) 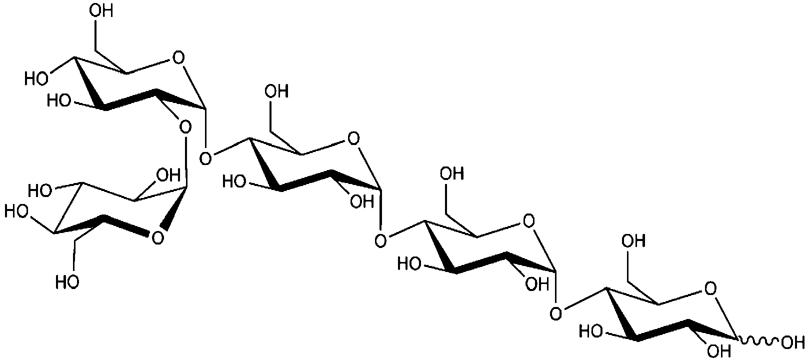 2-O-α-D-glucosyl-maltotetraose
(α-D-Glucopyranosyl-(1→2)-α-D-glucopyranosyl-(1→4)-α-D-glucopyranosyl-(1→4)-α-D-glucopyranosyl-(1→4)-D-glucopyranose) 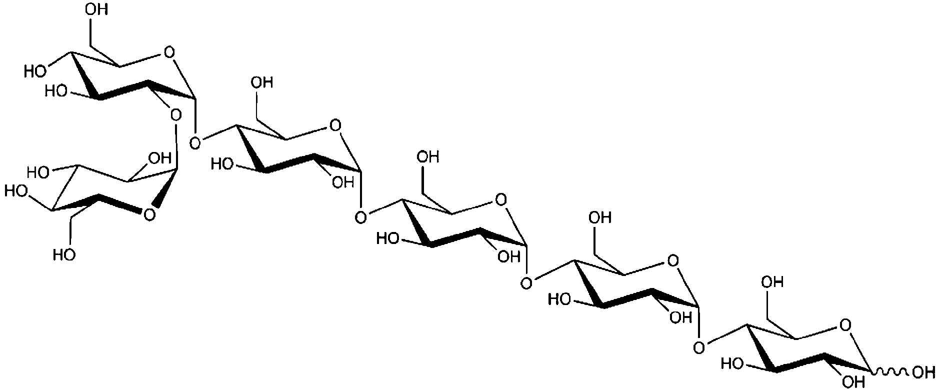 2-O-α-D-glucosyl-maltopentaose
(α-D-Glucopyranosyl-(1→2)-α-D-glucopyranosyl-(1→4)-α-D-glucopyranosyl-(1→4)-α-D-glucopyranosyl-(1→4)-α-D-glucopyranosyl-(1→4)-D-glucopyranose) 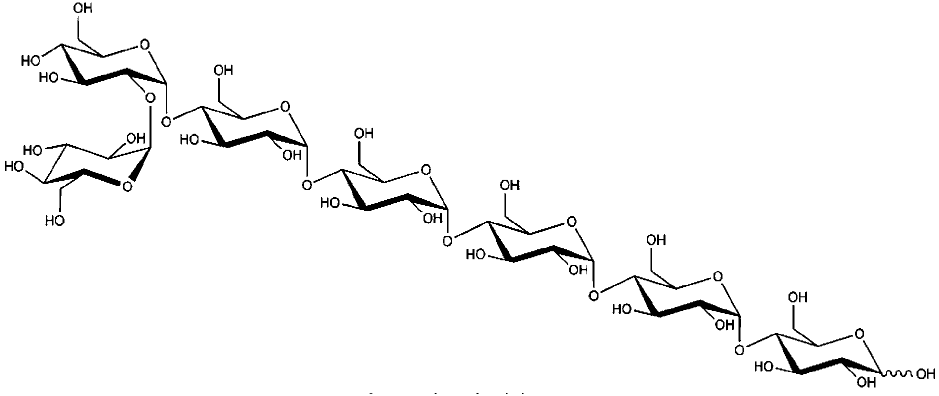 2-O-α-D-glucosyl-maltohexaose
(α-D-Glucopyranosyl-(1→2)-α-D-glucopyranosyl-(1→4)-α-D-glucopyranosyl-(1→4)-α-D-glucopyranosyl-(1→4)-α-D-glucopyranosyl-(1→4)-α-D-glucopyranosyl-(1→4)-D-glucopyranose)

15. A foodstuff, pet food, feed or personal care product comprising the composition according to any one of claims 1 to 14.
